# EUROPÄISCHE PATENTANMELDUNG

(11) **EP 2 583 646 A1**
(43) Veröffentlichungstag der Anmeldung: **24.04.2013**
(21) Anmeldenummer: 11405339.0
(22) Anmeldetag: 18.10.2011
(51) Int. Cl.: A61F 13/02

(54) **Verschlussvorrichtung zum Verschliessen von eröffneten Blutgefässen und Verwendung dieser Verschlussvorrichtung**

(71) Anmelder: VOSTRA-MED AG, 6330 Cham (CH)
(72) Erfinder: Harren, Ernst-Diethelm, 8570 Weinfelden (CH); Banthien, Felix, 78187 Geisingen (DE)
(74) Vertreter: EGLI-EUROPEAN PATENT ATTORNEYS

(57) **Zusammenfassung**

Eine ortsdefiniert aufklebbare Verschlussvorrichtung (1) zum Verschliessen von eröffneten Blutgefässen hat ein Verschlusselement (2) und einen Druckkörper (3), wobei das Verschlusselement (2) aus einem Material mit elastischen Eigenschaften ausgebildet ist. Die Verschlussvorrichtung (1) enthält ferner eine Vielzahl von Haltebändern (4), welche an distalen Enden des Verschlusselementes (2) anschliessen, wobei die Haltebänder (4) unelastische Eigenschaften haben; und zumindest ein Indikatorelement (8), welches mit dem Verschlusselement (2) verbunden ist. Die Vorteile bestehen in einem einfachen Aufbau der aufklebbaren Verschlussvorrichtung (1) sowie in der guten und auch für Laien einfachen Handhabbarkeit.

## Beschreibung

Die Erfindung betrifft eine Verschlussvorrichtung zum Verschliessen von eröffneten Blutgefässen nach Patentanspruch 1 und eine Verwendung dieser Verschlussvorrichtung nach Patentanspruch 16.

Die Erfindung betrifft insbesondere eine ortsdefiniert aufklebbare Verschlussvorrichtung zum Verschliessen von eröffneten Blutgefässen mit einem Druckkörper, der geeignet ist, mit einer Wunde in Berührung zu gelangen.

In diesem Bereich der medizinischen Anwendungen, insbesondere aber bei der Punktion von Gefässen, gibt es noch immer einen Bedarf, eine auch für einen medizinischen Laien einfach zu handhabende Verschlussvorrichtung zum Verschliessen von eröffneten Blutgefässen, im Folgenden auch Gefässverschluss genannt, zur Verfügung zu stellen, mit der nicht nur der anschliessende Blutaustritt bzw. Blutverlust in den freien Raum ausserhalb des Körpers, sondern auch der Blutaustritt in das Gewebe (Hämatombildung) verhindert wird, wobei gleichzeitig aber auch die lebenswichtige Blutzirkulation innerhalb des Körpers nach erfolgtem Anlegen der Verschlussvorrichtung nicht behindert werden soll.

In herkömmlicher Weise geschieht das Verschliessen von Punktionsstellen eines Blutgefässes derart, dass nach dem Eingriff auf die Gefässeinstichstelle ein Druck ausgeübt wird. Wegen der Gefahr der Hämatombildung darf der Druck nicht zu klein sein, wegen der Gefahr der Kreislaufunterbindung im betroffenen Körperteil aber auch nicht zu gross. Es gibt deshalb Lösungen, bei denen versucht wird, einen adäquaten Gegendruck, also einen dem Blutdruck entsprechenden Gegendruck, mittels einer durch das austretende Blut sich füllenden externen Blutkammer bzw. externen Druckkammer und in dieser Weise als Druckkörper wirkenden Verschlussvorrichtung, aufzubauen. Zur Unterstützung werden teilweise noch zusätzliche mechanische Druckkörper eingesetzt.

Ein Beispiel einer derartigen Verschlussvorrichtung ist in der DE 44 29 230 beschrieben. Diese Druckschrift zeigt einen aufklebbaren Punktionsverschluss mit einer Druckkammer und einer dehnbaren Druckwand. Dieser Punktionsverschluss wird bereits vor dem Punktieren der Gefässe aufgeklebt. Beim Punktieren werden die Druckwand und Druckkammer durchstochen.

Der aufklebbare Punktionsverschluss gemäss DE 44 29 230 hat allerdings den Nachteil, dass er im Aufbau, wegen der stets vorhandenen Druckkammer, relativ kompliziert ist. Zudem ist er in der Handhabung nicht ganz einfach, weil die zu punktierenden Gefässe stets durch das Verschlusselement und die dehnbare Druckwand der Druckkammer punktiert werden müssen.

Ein weiteres Beispiel eines Gefässverschlusses ist in der WO 2006/042430 A1 offenbart. Dieser Gefässverschluss enthält eine Verschlussschicht und einen Druckkörper, welche einstückig aus einem dehnbaren Material ausgebildet sind. Ferner ist eine Trägerschicht vorgesehen, welche grossflächig über eine Kleberschicht mit der Verschlussschicht verbunden ist. Über eine wiederrum mit der Trägerschicht verbundene Hautkleberschicht ist der Gefässverschluss im Bereich der Punktionsöffnung auf die Haut des Patienten aufklebbar. Es besteht ein Problem darin, dass der grossflächige, mehrlagige Aufbau hohe Kosten verursacht. Ein weiteres Problem besteht darin, dass der auf die Punktionsöffnung ausgeübte Druck in einigen Fällen zu hoch ist, so dass die Gefahr der Kreislaufunterbindung im betroffenen Körperteil besteht, und in einigen Fällen zu niedrig ist, so dass die Gefahr der Hämatombildung besteht.

Aufgabe der Erfindung ist es deshalb, eine verbesserte Verschlussvorrichtung zum Verschliessen von eröffneten Blutgefässen anzugeben.

Diese Aufgabe wird durch eine Verschlussvorrichtung zum Verschliessen von eröffneten Blutgefässen mit den Merkmalen des Patentanspruchs 1 gelöst.

Die Verschlussvorrichtung zum Verschliessen von eröffneten Blutgefässen enthält ein Verschlusselement und einen Druckkörper, wobei das Verschlusselement aus einem Material mit elastischen Eigenschaften ausgebildet ist. Erfindungsgemäss enthält die Verschlussvorrichtung ferner eine Vielzahl von Haltebändern, welche an distalen Enden des Verschlusselementes anschliessen, wobei die Haltebänder unelastische Eigenschaften haben; und zumindest ein Indikatorelement, welches mit dem Verschlusselement verbunden ist.

Die erfindungsgemässe Verschlussvorrichtung zum Verschliessen eines eröffneten Blutgefässes gestattet es überraschend einfach, einen individuell durch Zugkraft auf die Verschlussvorrichtung einstellbaren Druck auf die Punktionsöffnung anzulegen. Die hierzu nötige Zugkraft lässt sich, noch bevor die Verschlussvorrichtung aufgeklebt wird, anhand des Indikatorelements ablesen. Zur besseren Ablesbarkeit sind zumindest zwei Indikatorelemente bereitgestellt. Mit anderen Worten, ist es möglich, anhand der veränderlichen Form bzw. Geometrie der Indikatorelemente, welche durch veränderliche Zugkraft auf die Verschlussvorrichtung hervorgerufen wird, einen Rückschluss auf den jeweiligen Druck auf die Punktionsöffnung zu ziehen, wenn die Verschlussvorrichtung in dem (unter Zugkraft gesetzten) Zustand aufgeklebt wird.

Eine hohe Präzision in der Ablesbarkeit der Indikatorelemente wird überraschend einfach und zuverlässig dadurch erreicht, indem das Verschlusselement elastische Eigenschaften hat und die Haltebänder wiederum unelastische Eigenschaften haben. Es ist hierbei zu erwähnen, dass als Haltebänder jeweils Ausläufer an den Enden des Verschlusselements definiert sind. An diesen Haltebändern ergreift der Benutzer die Verschlussvorrichtung, beispielsweise zwischen Zeigefinger und Daumen, und übt durch Strecken der Verschlussvorrichtung eine Zugkraft auf die Verschlussvorrichtung aus. Diese Zugkraft wird wiederrum fast ausschliesslich durch das Material des Verschlusselementes aufgenommen, welches erfindungsgemäss elastische Eigenschaften besitzt. Die erfindungsgemässe Verschlussvorrichtung erlaubt eine einfache und sichere Handhabung. Ausserdem kann der auf die Punktionsöffnung angelegte Druck durch Ablesen der Indikatorelemente individuell und anwendungsspezifisch eingestellt werden. Somit ist immer der korrekte Druck auf die Punktionsstelle einstellbar. Zudem ist der Aufbau der erfindungsgemässen Verschlussvorrichtung sehr einfach, wodurch die Herstellungskosten gesenkt werden.

Vorzugsweise sind das Verschlusselement und die Haltebänder einstückig ausgebildet. Die Verschlussvorrichtung ist hierbei mit geeignet langen Haltebändern versehen. Neben der guten Handhabbarkeit soll damit auch eine genügende Haftung auf der Haut erreicht werden, um den zur Blutstillung nötigen Gegendruck aufbauen zu können. Die Geometrie der Verschlussvorrichtung ist nicht an eine solche Form gebunden. Andere Formen sind ebenso denkbar, wie beispielsweise kreisförmige Patches. Allerdings, wie zuvor erwähnt, muss das Verschlusselement eine elastische Eigenschaft aufweisen und müssen die Haltebänder eine unelastische Eigenschaft aufweisen. Hierdurch lässt sich die notwendige Zugkraft anhand der Indikatorelemente ablesen, um somit einen individuell notwendigen Druck auf die Punktionsöffnung anlegen zu können.

Alternativ vorzugsweise sind das Verschlusselement und die Haltebänder mehrstückig ausgebildet. In dieser Ausführungsform können das Verschlusselement aus einem elastischen Material und die Vielzahl von Haltebändern aus einem unelastischen Material durch geeignete Verbindungsverfahren miteinander verbunden werden.

Vorzugsweise sind das Verschlusselement und/oder der Druckkörper und/oder das Indikatorelement einstückig ausgebildet. Durch die Ausformung dieser Elemente aus einem einzigen Material sind hohe Kosteneinsparungen erzielbar. Zudem sind hohe Stückzahlen in kurzer Zeit herstellbar.

Vorzugsweise ist das zumindest eine Indikatorelement dazu ausgelegt, mit zunehmender Streckung durch Anlegen einer Zugkraft auf das Verschlusselement eine veränderliche Geometrie anzunehmen. Hierdurch kann in Ansprechen auf einen gewünschten Druck, welcher auf die Punktionsöffnung anzulegen ist, eine derartige Zugkraft auf die Enden der Verschlussvorrichtung angelegt werden, bis die Indikatorelemente eine hierzu notwendige (definierte) Geometrie annehmen. Sobald der Benutzer die hierzu definierte Geometrie der Indikatorelemente optisch abliest bzw. erkennt, ist die korrekte Zugkraft angelegt und kann die mit dieser Zugkraft gestreckte Verschlussvorrichtung auf die Haut des Patienten angebracht werden. Hierdurch ist es vorteilhafterweise möglich, mit einfachen Mitteln einen individuellen Druck auf die Punktionsöffnung anzulegen.

Vorzugsweise nimmt das zumindest eine Indikatorelement in einem unbelasteten Zustand des Verschlusselements jeweils die Geometrie einer ovalen Form an, welche mit zunehmender Streckung des Verschlusselements in eine zunehmend kreisförmige Form übergeht und mit weiter zunehmender Streckung in eine zunehmend ovale Form übergeht. Durch die Ausgestaltung der Indikatorelemente in ovaler Form ist eine besonders zuverlässige Einstellung der jeweils notwendigen Zugkraft auf die Verschlussvorrichtung durch Inaugenscheinnahme der sich veränderlichen Form möglich. In Abhängigkeit von der medizinischen Indikation stellt sich eine veränderte Geometrie ein. Durch Strecken der Verschlussvorrichtung wird die anfangs ovale Form der Indikatorelemente zunehmend rund. Durch ein Strecken der Verschlussvorrichtung über diesen gestreckten Zustand hinaus ändert sich die Geometrie der Indikatorelemente von der runden Form auf eine ovale Form. Durch Ablesen der veränderlichen Form kann die nötige Zugkraft einfach und zuverlässig definiert werden.

Vorzugsweise enthält die Verschlussvorrichtung ferner ein flüssigkeitsabsorbierendes Element, welches umfangsseitig um den Druckkörper angeordnet ist. Dieses flüssigkeitsabsorbierende Element saugt möglicherweise aus der Nadel austretendes Blut auf. Die Verschlussvorrichtung hat auch unter hygienischen Aspekten Vorteile gegenüber bekannten und gängigen Lösungen. Die Verschlussvorrichtung ist als steriles Einwegprodukt vorgesehen, um insbesondere auch Kreuzinfektionen auszuschliessen.

Vorzugsweise enthält das flüssigkeitsabsorbierende Element einen Wirkstoff mit schmerzstillenden und/oder blutstillenden und/oder antiallergischen Wirkstoffen. Hierdurch wird eine gute Verträglichkeit auch bei langandauernder Applikation der Verschlussvorrichtung am Patienten gewährleistet.

Vorzugsweise ist jeweils in dem Bereich der Haltebänder, auf einer Seite der Verschlussvorrichtung, auf welcher der Druckkörper bereitgestellt ist, eine Verstärkungsschicht angebracht, welche aus einem Material mit unelastischen Eigenschaften ausgebildet ist. Mittels dieser Verstärkungsschicht werden den Haltebändern die notwendigen unelastischen Eigenschaften vermittelt. Hierbei ist zu verstehen, dass unter einem Material mit unelastischen Eigenschaften ein Material zu verstehen ist, das keine Erstreckung in Längsals auch in Querrichtung zulässt. Wie anhand der vorherigen Beschreibung des Anwendungsbereiches zu verstehen, ist selbstverständlich eine derartige Verformbarkeit des Materials gegeben, welche ein Anschmiegen und Verkleben der Haltebänder an auch nicht ebenen Körperbereichen des Menschen zulässt.

Vorzugsweise ist die Verstärkungsschicht mittels eines Klebemittels jeweils an einer Seite mit dem Bereich der Haltebänder verbunden und auf der weiteren Seite mit einer Hautkleberschicht bereitgestellt. Mit Hilfe der Hautkleberschicht wird die Verschlussvorrichtung mittels Klebekraft derart an der Haut des Patienten fixiert, dass der Druckkörper die Punktionsöffnung hierdurch zuverlässig verschliesst.

Vorzugsweise ist die Hautkleberschicht von einer abziehbaren Schutzfolie bedeckt. Hierdurch wird die Hautkleberschicht geschützt und kann somit eine langandauernde Klebekraft der Hautkleberschicht gewährleistet werden. Dem Anlegen der Verschlussvorrichtung auf die Hautfläche des Patienten geht lediglich ein Abziehen der Schutzfolie von der Hautkleberschicht voraus.

Vorzugsweise bestehen das Verschlusselement und/oder der Druckkörper und/oder die Haltebänder und/oder das Indikatorelement aus Silikon, Naturkautschuk, Kunstkautschuk, Gummi, Latex, Hydrogel, polymerem Kunststoff oder einer Kombination dieser Materialien. Wie zuvor beschrieben, besitzen die Materialien des Verschlusselements eine hohe Elastizität und weisen eine gewünscht hohe Rückstellkraft auf. Insbesondere ist Silikon wegen der hervorragenden medizinischen Eignung ein bevorzugtes Material für die Verschlussvorrichtung. Ein Vorteil in der Auswahl von Silikon besteht ferner darin, dass die vorhandene ausserordentlich hohe Dehnfähigkeit in der flächigen Ausdehnbarkeit begrenzt und stabilisiert ist. Dies ist notwendig, um den zur Blutstillung notwendigen, individuell einstellbaren Gegendruck aufbringen zu können.

Vorzugsweise sind das Verschlusselement und/oder der Druckkörper und/oder die Haltebänder und/oder das Indikatorelement und/oder die Verstärkungsschicht transparent oder annähernd transparent. Hierdurch wird das Aufbringen der Verschlussvorrichtung als Punktionsverschluss an korrekter Position erleichtert.

Vorzugsweise sind die Haltebänder wasserdampfdurchlässig. Hierdurch wird die Trageeigenschaft der Verschlussvorrichtung verbessert.

Vorzugsweise sind die Haltebänder perforiert. Durch die Perforierungen kann Wasserdampf schnell abgeführt werden, was die Trageeigenschaft der Verschlussvorrichtung verbessert.

Die Anfangs genannte Aufgabe wird ebenfalls durch eine Verwendung einer Verschlussvorrichtung nach einem der Ansprüche 1 bis 15 zum Verschliessen eines eine Punktionsöffnung aufweisenden Blutgefässes gelöst. Durch diese Verwendung wird ein eröffnetes Blutgefäss auf überraschend einfache Weise, individuell mit einstellbarem Druck auf die Punktionsöffnung, geschlossen.

Im Folgenden wird die erfindungsgemässe Verschlussvorrichtung anhand der Zeichnung detailliert beschrieben.

In der Zeichnung zeigen:
- Fig. 1: einen Längsschnitt durch eine ortsdefiniert aufklebbare Verschlussvorrichtung;
- Fig. 2: eine Ansicht auf die Verschlussvorrichtung gemäss Fig. 1;
- Fig. 3: eine schematische Skizze zur Anwendung der Verschlussvorrichtung; und
- Fig. 4a-c: schematische Ansichten der Verschlussvorrichtung bei unterschiedlichen Streckungen.

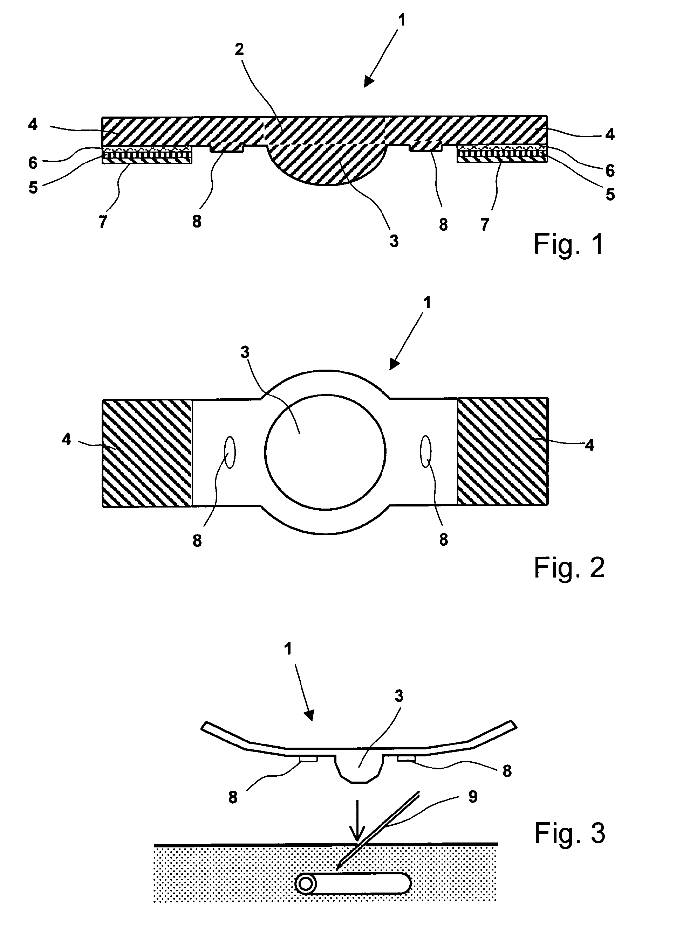

Die Fig. 1 zeigt beispielhaft einen nicht-massstäblichen Längsschnitt durch eine ortsdefiniert aufklebbare Verschlussvorrichtung 1, und Fig. 2 zeigt hierzu eine Ansicht von unten auf die Verschlussvorrichtung 1 gemäss Fig. 1. Die Verschlussvorrichtung 1 enthält ein Verschlusselement 2 und einen Druckkörper 3, welche in diesem Beispiel einstückig aus einem Material mit elastischen Eigenschaften, beispielsweise Silikon, ausgebildet sind. Der Druckkörper 3 ist ein etwa linsenförmiges Gebilde und dient zum Anlegen einer Druckkraft zum Verschliessen einer Punktionsöffnung (nicht gezeigt).

Ferner sind zwei Haltebänder 4 als Ausläufer des Verschlusselements 2 an distalen Enden davon bereitgestellt. Die Haltebänder 4 haben unelastische Eigenschaften. Beispielsweise können die Haltebänder 4 einstückig mit dem Verschlusselement 2 ausgebildet sein. Um den Haltebändern 4 die unelastischen Eigenschaften zu vermitteln, ist beispielsweise eine Verstärkungsschicht 5 auf die Haltebänder 4 angebracht. Diese Verstärkungsschicht 5 ist wiederum aus einem Material mit unelastischen Eigenschaften ausgebildet. Die Verstärkungsschicht 5 kann mittels eines herkömmlichen (technischen) Klebemittels 6 auf die Haltebänder 4 aufgeklebt werden. In einem Fall, bei welchem die Haltebänder 4 aus Silikon hergestellt sind, kann es notwendig sein, die Kontaktfläche mit dem Klebemittel 6 in geeigneter Weise vorzubereiten. Um den dauerhaften Kontakt von Silikon mit dem verwendeten Klebemittel 6 zu verbessern, kann deshalb eine Oberflächenbehandlung, wie z.B. eine Plasma-, Corona-, nasschemische-oder sonstige Behandlung, vorgesehen sein.

Alternativ kann das gemeinsame Material, aus welchem das Verschlusselement 2 und die Haltebänder 4 einstückig ausgebildet sind, derart durch beispielsweise gezielte Aushärte-Verarbeitungsabläufe bearbeitet werden, dass lediglich nur das Material im Bereich der Haltebänder 4 keine Dehnung zulässt, während die restlichen Bereiche weiterhin dehnbar sind.

Obwohl in der Zeichnung nicht gezeigt, können die Haltebänder 4 und das Verschlusselement 2 aus unterschiedlichen Materialien bestehen, welche miteinander verbunden sind. In diesem Beispiel kann das Verschlusselement 2 aus Silikon bestehen und können die Haltebänder 4 aus Polyethylen (PE) bestehen.

An der Unterseite der Verstärkungsschicht 5 ist eine Hautkleberschicht 7 zum Aufkleben auf die Haut bereitgestellt. Die Hautkleberschicht 7 hat vorzugsweise hautkleberspezifische Eigenschaften. Schliesslich kann die Hautkleberschicht 7 zusätzlich noch mit einer abziehbaren Schutzfolie versehen sein (nicht dargestellt).

Ferner enthält die Verschlussvorrichtung 1 in dem Bereich des Verschlusselementes 2 zwei Indikatorelemente 8, welche mit dem Verschlusselement 2 verbunden sind. In einer Ausführungsform sind die Indikatorelemente 8 einstückig mit dem Verschlusselement 2 ausgebildet. Die Indikatorelemente 8 sind hierbei beidseitig vom Druckkörper 3, vorzugsweise bei gleichen Abständen, angeordnet. Die Indikatorelemente 8 sind dazu ausgelegt, eine mit zunehmender Streckung durch Anlegen einer Zugkraft auf die Verschlussvorrichtung 1 (und somit auf das Verschlusselement 2) veränderliche Geometrie anzunehmen, welche Veränderlichkeit in Relation zur Streckung und somit zur Zugkraft steht.

Das Verschlusselement 2, der Druckkörper 3, die Haltebänder 4, die Indikatorelemente 8 und die Verstärkungsschicht 5 sind vorzugsweise transparent oder annähernd transparent ausgebildet, um das Aufbringen der ortsdefiniert aufklebbaren Verschlussvorrichtung 1 zu erleichtern.

Wie bereits erwähnt, können das Verschlusselement 2, der Druckkörper 3, die Haltebänder 4 und die Indikatorelemente 8 aus einem einstückigen Silikonmaterial bestehen. Neben der ausserordentlichen Dehnbarkeit, weist Silikon auch eine erwünschte hohe Rückstellkraft auf. Es können aber auch andere Materialien, wie z.B. Naturkautschuk, Kunstkautschuk, Gummi, Latex, Hydrogel, polymerer Kunststoff oder eine Kombination dieser Materialien, verwendet werden. Alternativ können die Haltebänder aus Polyethylen (PE) hergestellt sein.

Der Druckkörper 3 hat im Normalfall (beispielsweise nach einer Untersuchung und/oder Behandlung, usw.) direkten Hautkontakt. Deshalb sind für die verwendeten Materialien geeignete medizinische Eigenschaften, vor allem hinsichtlich der Haut- und Körperverträglichkeit, erforderlich.

Anhand von Fig. 3 wird die Verwendung der ortsdefiniert aufklebbaren Verschlussvorrichtung 1 zum Verschliessen eines eröffneten Gefässes beschrieben.

Bevor eine Nadel oder Kanüle 9 aus der Einstichstelle entnommen wird, wird die entsprechende Körperstelle auf herkömmliche Weise gereinigt und desinfiziert. Anschliessend werden die Schutzfolien (in den Figuren nicht dargestellt) von beiden Haltebändern der Verschlussvorrichtung 1 abgezogen. Die Verschlussvorrichtung 1 wird dann auf die betreffende Körperstelle mittels der Haltebänder derart angelegt und fixiert, dass der Druckkörper 3 später bei der vollständigen Applikation der Verschlussvorrichtung 1 präzise über die Einstichstelle zum Liegen kommt, wie in Fig. 3 durch den Pfeil angedeutet. Anschliessend wird die Nadel oder Kanüle 9 entnommen. Als Einsatzgebiete für die Verschlussvorrichtung 1 seien genannt: Dialyse, vor dem Entfernen der Fistelnadel; Allgemeinmedizin, vor dem Entfernen der Verweilkanüle; Radiologie, vor dem Entfernen des Katheders; Minimalinvasive Chirurgie, vor dem Entfernen des Endoskops und/oder sonstiger Untersuchungsgerätschaften.

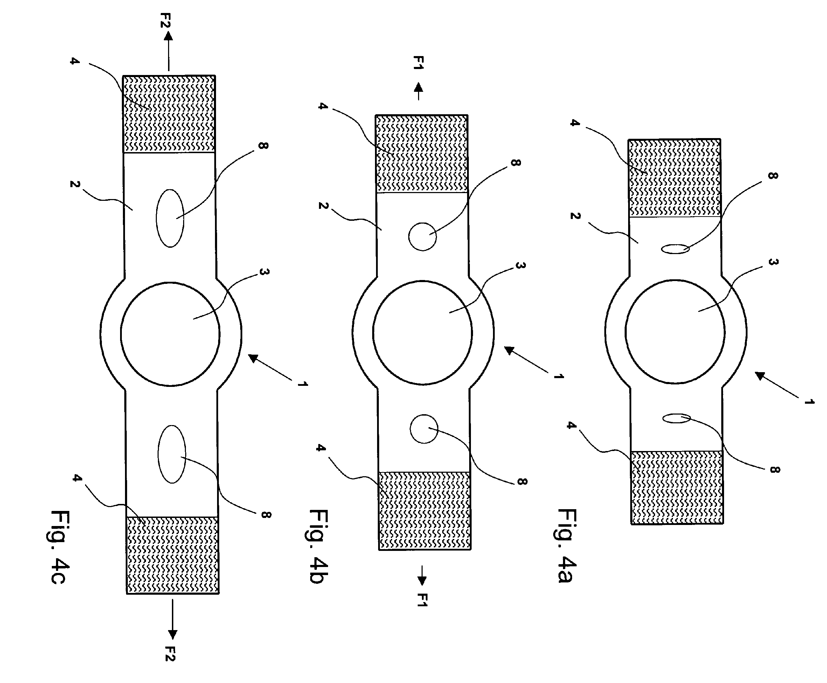

In Figuren 4a-c sind schematische Ansichten der Verschlussvorrichtung 1 bei unterschiedlichen Streckungen dieser gezeigt. Die Indikatorelemente 8 sind in dieser Ausführungsform einstückig mit dem Verschlusselement 2 ausgebildet. Die Indikatorelemente 8 können beispielsweise in Relation zum Verschlusselement 2 als Aussparung oder Hervorhebung ausgeführt sein. In einem weiteren Beispiel können die Indikatorelemente 8 mittels Materialabtrag bzw. Materialauftrag an lediglich der Kontur der Indikatorelemente 8 ausgeführt werden. Es können auch mehr als zwei Indikatorelemente 8 vorgesehen werden.

Im ursprünglichen, unbelasteten Zustand der Verschlussvorrichtung 1 (siehe Fig. 4a) sind die Indikatorelemente 8 mit der Geometrie einer ovalen Form ausgebildet. Das Zentrum der Indikatorelemente 8 sollte jeweils in einem Abschnitt des Verschlusselements 2 in der Mitte zwischen dem Druckkörper 3 und dem Ansatz zum Halteband 4 zum Liegen kommen. Es sind auch andere Formen als die ovale Form für die Indikatorelemente 8 denkbar.

Durch ein Strecken der Verschlussvorrichtung 1 durch Anlegen von jeweils einer Zugkraft F1 an die Haltebänder 4 wird aufgrund der elastischen Eigenschaft des Materials des Verschlusselements 2 lediglich dieses Element in die Länge gestreckt. Durch diese Streckung werden auch die Indikatorelemente 8 gestreckt, mit dem Ergebnis, dass deren ovale Form zunehmend in eine kreisförmige Form übergeht, wie in Fig. 4b gezeigt.

Durch Erhöhen der Zugkräfte von F1 auf F2, geht die kreisförmige Form in eine ovale Form über, wie in Fig. 4c gezeigt. Durch Betrachten der jeweiligen Form der Indikatorelemente 8 ist der Benutzer dazu in der Lage, einen Rückschluss auf die jeweilige Zugkraft zu ziehen. In Ansprechen hierauf ist der Benutzer somit dazu in der Lage, den Druck definieren zu können, welchen der Druckkörper 3 einer derart gestreckten Verschlussvorrichtung 1 an die Einstichstelle anlegt, sobald die derart gestreckte Verschlussvorrichtung 1 auf die Haut des Patienten angeklebt ist. Somit kann der Druck schnell, einfach und anwendungsspezifisch definiert werden.

## Patentansprüche

1. Verschlussvorrichtung (1) zum Verschliessen eines eröffneten Blutgefässes, mit einem Verschlusselement (2) und einem Druckkörper (3), wobei das Verschlusselement (2) aus einem Material mit elastischen Eigenschaften ausgebildet ist;
**gekennzeichnet durch**
eine Vielzahl von Haltebändern (4), welche an distalen Enden des Verschlusselementes (2) anschliessen, wobei die Haltebänder (4) unelastische Eigenschaften haben; und
zumindest ein Indikatorelement (8), welches mit dem Verschlusselement (2) verbunden ist.

2. Verschlussvorrichtung (1) nach Patentanspruch 1, **dadurch gekennzeichnet, dass** das Verschlusselement (2) und die Haltebänder (4) einstückig ausgebildet sind.

3. Verschlussvorrichtung (1) nach Patentanspruch 1, **dadurch gekennzeichnet, dass** das Verschlusselement (2) und die Haltebänder (4) mehrstückig ausgebildet sind.

4. Verschlussvorrichtung (1) nach einem der Patentansprüche 1 bis 3, **dadurch gekennzeichnet, dass** das Verschlusselement (2) und/oder der Druckkörper (3) und/oder das zumindest eine Indikatorelement (8) einstückig ausgebildet sind.

5. Verschlussvorrichtung (1) nach einem der Patentansprüche 1 bis 4, **dadurch gekennzeichnet, dass** das zumindest eine Indikatorelement (8) dazu ausgelegt ist, mit zunehmender Streckung durch Anlegen einer Zugkraft auf das Verschlusselement (2) eine veränderte Geometrie anzunehmen.

6. Verschlussvorrichtung (1) nach Patentanspruch 5, **dadurch gekennzeichnet, dass** das zumindest eine Indikatorelement (8) in einem unbelasteten Zustand des Verschlusselements (2) jeweils die Geometrie einer ovalen Form annimmt, welche mit zunehmender Streckung des Verschlusselements (2) in eine zunehmend kreisförmige Form übergeht und mit weiter zunehmender Streckung in eine zunehmend ovale Form übergeht.

7. Verschlussvorrichtung (1) nach einem der Patentansprüche 1 bis 6, ferner **gekennzeichnet durch** ein flüssigkeitsabsorbierendes Element, welches umfangsseitig um den Druckkörper (3) angeordnet ist.

8. Verschlussvorrichtung (1) nach Patentanspruch 7, **dadurch gekennzeichnet, dass** das flüssigkeitsabsorbierende Element einen Wirkstoff mit schmerzstillenden und/oder blutstillenden und/oder antiallergischen Wirkstoffen enthält.

9. Verschlussvorrichtung (1) nach einem der Patentansprüche 1 bis 8, **dadurch gekennzeichnet, dass** jeweils in dem Bereich der Haltebänder (4), auf einer Seite der Verschlussvorrichtung (1), auf welcher der Druckkörper (3) bereitgestellt ist, eine Verstärkungsschicht (5) angebracht ist, welche aus einem Material mit unelastischen Eigenschaften ausgebildet ist.

10. Verschlussvorrichtung (1) nach Patentanspruch 9, **dadurch gekennzeichnet, dass** die Verstärkungsschicht (5) mittels eines Klebemittels (6) jeweils an einer Seite mit dem Bereich der Haltebänder (4) verbunden ist und auf der weiteren Seite mit einer Hautkleberschicht (7) bereitgestellt ist.

11. Verschlussvorrichtung (1) nach Patentanspruch 10, **dadurch gekennzeichnet, dass** die Hautkleberschicht (7) mit einer abziehbaren Schutzfolie bedeckt ist.

12. Verschlussvorrichtung (1) nach einem der Patentansprüche 1 bis 11, **dadurch gekennzeichnet, dass** das Verschlusselement (2) und/oder der Druckkörper (3) und/oder die Haltebänder (4) und/oder das zumindest eine Indikatorelement (8) aus Silikon, Naturkautschuk, Kunstkautschuk, Gummi, Latex, Hydrogel, polymerem Kunststoff oder einer Kombination dieser Materialien bestehen.

13. Verschlussvorrichtung (1) nach einem der Patentansprüche 1 bis 12, **dadurch gekennzeichnet, dass** das Verschlusselement (2) und/oder der Druckkörper (3) und/oder die Haltebänder (4) und/oder das zumindest eine Indikatorelement (8) und/oder die Verstärkungsschicht (5) transparent oder annähernd transparent sind.

14. Verschlussvorrichtung (1) nach einem der Patentansprüche 1 bis 13, **dadurch gekennzeichnet, dass** die Haltebänder (4) wasserdampfdurchlässig sind.

15. Verschlussvorrichtung (1) nach einem der Patentansprüche 1 bis 14, **dadurch gekennzeichnet, dass** die Haltebänder (4) perforiert sind.

16. Verwendung einer Verschlussvorrichtung (1) nach einem der Ansprüche 1 bis 15 zum Verschliessen eines eine Punktionsöffnung aufweisenden Blutgefässes.
